# EUROPEAN PATENT SPECIFICATION

(11) **EP 1 982 656 B1**
(45) Date of publication and mention of the grant of the patent: **15.08.2012**
(21) Application number: 08251145.2
(22) Date of filing: 28.03.2008
(51) Int. Cl.: A61B 17/04, A61B 17/08

(54) **Systems for approximating surfaces**
Systeme zur Oberflächenapproximation
Systèmes pour l'approximation de surfaces

(30) Priority: 19.04.2007 US 788767
(43) Date of publication of application: 22.10.2008
(73) Proprietor: The Invention Science Fund I, LLC, Bellevue, WA 98004 (US)
(72) Inventor: Bangera, Mahalaxmi Gita, Renton, WA 98059 (US); Boyden, Edward S., Cambridge, MA 02142 (US); Hyde, Roderick A., Redmond, WA 98052 (US); Ishikawa, Muriel Y., Livermore, CA 94550-4921 (US); Jung, Edward K. Y., Bellevue, WA 98005-1403 (US); Leuthardt, Eric C., St. Louis, MO 63130 (US); Rivet, Dennis J. II, Portsmouth, VA 23704 (US); Smith, Michael A., Phoenix, AZ 85050 (US); Sweeney, Elizabeth A., Seattle, WA 98133 (US); Tegreene, Clarence T., Bellevue, WA 98004-2834 (US); Wood, Lowell L. Jr., Bellevue, WA 98004 (US); Wood, Victoria Y. H., Livermore, CA 94550-4921 (US)
(74) Representative: Harris, Ian Richard

(56) References cited:
- EP-A- 0 702 934
- WO-A-2005/020802
- WO-A-2006/026634
- WO-A-2006/027678
- US-A- 3 926 193

## Description

W02005/020802 discloses a T-fastener delivery device. The device includes a belt and a pusher rod. The belt includes a plurality of slots for holding a plurality of T-bars substantially parallel to one another in a side-by-side arrangement; and the belt is moveable such that the slots can be sequentially aligned with the pusher rod. Also disclosed are methods for creating a gastric pouch in the stomach of an obese patient and methods for reducing lower esophageal sphincter distraction with gastric filling in a subject. Suturing systems are also disclosed. One suturing system includes a plurality of T-fasteners and a continuous suture connecting the plurality of T-fasteners. The T-fastener comprises a cylindrical bar having a filament loop attached thereto substantially at the cylindrical bar's axial midpoint, and the continuous suture slidably passes through the T-fasteners' filament loops. Another suturing system includes a plurality of T-fasteners; and a continuous suture connecting the plurality of T-fasteners. The T-fastener comprises two cylindrical bars connected by a filament, and the continuous suture slidably passes through a loop formed by the T-fasteners' filaments.

EP 0,702,934 discloses a surgical attachment device. The device has an elastomeric member which may be bioabsorbable. A tissue engagement means such as a hook is mounted to the elastomeric member. The tissue engagement means may also be bioabsorbable. The device provides tensioned fastening.

US 3,926,193 discloses a sutureless closure device for drawing the edges of an incision together. The device comprises first and second surgical tape members for application to opposite sides of a wound or incision. The first tape member carries tie member anchors, and the second tape member carries tie member receiving slides. Tie members connect respective anchors and slides, causing the two tape members to be biased together, which holds the incision closed. The device then functions as a splint to protect the healing wound against disruptive forces.

WO2006/026634 discloses tissue closure devices for closing a tissue opening, including one or more closure components, each closure component having a first member and a second member, each of the first and second members having a first surface that adheres to a tissue surface proximate to the tissue opening, each of the first and second members having a second surface substantially orthogonal to the first surface, and each of the first and second members having a transitional region between the first surface and the second surface which is contoured to evert an edge of the tissue opening upon the drawing together of the first and second members.

WO2006/027678 describes a system of non-reactive components for moving or for moving and stretching plastic tissue that exerts a relatively constant dynamic force over a variety of distances and geometries, that is easily adjustable, and is self-adjusting. This system includes a "button anchor system" for moving tissue, particularly including deep fascia and muscle layers of the abdominal or thoracic cavity wall, in surgical, post surgical, and post traumatic reconstruction where the wound margins are beyond a distance that permits normal re-approximation. Button anchor assemblies allow re-approximation of severely retracted abdominal wall and full thickness thoracic wounds where a closure force is required to be applied to the sub-dermal layers. The systems allows for such a force to be applied and externally controlled during treatment. A skin-contacting surface of a pad of the system may be coated or treated to provide antimicrobial properties.

### SUMMARY

The invention is defined in the claims.

In one embodiment, a system for maintaining approximation of two surfaces includes a first anchor configured to adhere to a first surface that includes tissue, a second anchor configured to adhere to a second surface (which may be tissue or non-tissue), and an approximation mechanisms configured to operably link and maintain the first and second anchors in a predetermined spatial relationship, wherein at least one of the first and second anchors includes a material having a therapeutic property selected from the group consisting of cell growth promoters, cell growth inhibitors, cytokines, healing promoters, antibiotics, clotting modulators, anti-inflammatory agents, and anti-scarring agents.

The approximation mechanisms may include a filament configured to maintain a tensile force tending to resist relative separation of the first and second anchors. The filament may be, for example, a suture (*e.g*., a shape-memory suture and/or a biodegradable suture), an elastomer, a cord, or a cable tie. The system may include a third anchor configured to adhere to a third surface, where the filament is configured to operably link and maintain the first, second, and third anchors in a predetermined relationship. The first and/or second anchors may be threaded onto the filament, and/or may include an opening configured to receive the filament (*e.g*., an eyelet, channel, and/or hook). The approximation mechanism may include a securing member configured to attach to each of the first and second anchors (*e.g*., a securing member configured to be inserted into openings in each of the first and second anchors, and/or a securing member including a conformable rod, a substantially straight rod, and/or a rod having a nonlinear shape). The approximation mechanism may include a stabilizing member (*e.g*., a flexible strip, a tape, and/or a flexible sheet) configured to support the first and second anchors in a predetermined relative position. The first and second anchors may be affixed to the stabilizing member, and/or may be configured to be coupled together via a coupling mechanism (*e.g*., a mechanism including a magnet, a mechanical fastener, an adhesive, and/or surfaces that adhere together by van der Waals forces). The approximation mechanism may be integral to at least one of the first and second anchors, such as a magnet that attaches the anchors together, a mechanical fastener (*e.g*., a tongue-in-groove connector, a retaining pin, a screw, a draw latch, a cable tie, and/or a hook and loop fastener) that fastens the anchors together reversibly or irreversibly, surfaces that adhere together by van der Waals forces, and/or an adhesive. The first anchor may be configured to adhere to the first surface via an adhesive, by penetrating tissue, by grasping, and/or by friction. Either or both of the anchors may include a surface-adherent portion configured to adhere to a surface and an engagement portion configured to engage the approximation mechanism. The surface-adherent portion and the engagement portion may be separate, separable, or integral, and may be configured to mechanically lock together, to be glued together with an adhesive, and/or to couple together with a fastener (*e.g*., a tongue-in-groove connector, a retaining pin, a screw, a draw latch, a cable tie, and/or a hook and loop fastener). The engagement portion may include an opening configured to accept at least a portion of the approximation mechanism, such as a loop, channel, and/or hook, and the approximation mechanism may include a filament configured to pass through the opening. Either or both of the anchors may include biocompatible material, biodegradable material, nonbiodegradable material and/or antimicrobial material. The system may include a third anchor configured to adhere to a third surface, wherein the approximation mechanism is configured to operably link and maintain the first, second, and third anchors in a predetermined spatial relationship. The second surface may include an organ for transplant, or an implant device such as a stent, a replacement heart valve, a breast implant, a cerebral stimulator, an endosseous implant, a bone growth matrix, an electrode, an aneurysm coil, and/or a graft.

An embodiment can provide a system for approximating body tissue includes a first anchor configured to adhere to body tissue, a second anchor configured to adhere to body tissue, and a traction member configured to draw the first anchor towards the second anchor. The first and second anchors and traction member are arranged to approximate body tissue upon drawing of the first anchor towards the second anchor. At least one of the first and second anchors includes a material having a therapeutic property selected from the group consisting of cell growth promoters, cell growth inhibitors, cytokines, healing promoters, antibiotics, clotting modulators, anti-inflammatory agents, and anti-scarring agents.

The traction member may be a suture, such as a shape-memory polymer suture, which may optionally be biodegradable. The anchors may be configured to adhere to tissue including, but not limited to, skin, blood vessel, muscle tissue, facial planes, dura, dermis, a visceral tube (*e.g*., an esophagus, a trachea, a respiratory tract, a stomach, a small intestine, a large intestine, a rectum, a ureter, and/or a vas deferens), and/or bone. The anchors may adhere by an adhesive, by penetrating tissue, and/or by friction. The first and second anchors may be configured to adhere to different body tissues. At least one of the first and second anchors may be configured to adhere by an adhesive, by penetrating tissue, and/or by friction. At least one of the anchors may include a tissue-adherent portion configured to adhere to body tissue, and an engagement portion configured to engage the traction member for drawing together of the anchors, in which case the tissue-adherent portion and the engagement portion may be separate, separable, and/or integral. The engagement portion may include a channel, loop, and/or hook configured to accept the traction member. The engagement portion may be attached to the traction member, for example by being threaded on a traction member including a flexible cord such as a suture. The anchor may include a fastener (*e.g*., a tongue-in-groove connector, a retaining pin, a screw, a draw latch, a cable tie, and/or a hook and loop fastener) configured to couple together the tissue-adherent portion and the engagement portion, and/or the tissue-adherent portion and the engagement portion may be configured to mechanically lock together and/or to be glued together with an adhesive. The anchors may include a biocompatible material, a biodegradable material, a nonbiodegradable material and/or an antimicrobial material

An embodiment can provide a system for approximating tissue includes a plurality of body-adherent tissue anchors and a securing member configured to hold the tissue anchors in a selected relative alignment. For example, the tissue anchors may each include an opening, and the securing member (*e.g*., a suture, a conformable rod, a substantially straight rod, and/or a rod having a nonlinear shape) may be configured to hold the tissue anchors by insertion of a portion of the member through each opening of the tissue anchors. The securing member may be configured to operably link to a first one of the plurality of body-adherent tissue anchors through a first connectivity mechanism, and to a second of the plurality of body-adherent tissue anchors through a second connectivity mechanism. (*e.g*., by insertion of at least a portion of a tissue anchor into an opening pointing the securing member, or via a mechanism such as a tongue-in-groove connector, a retaining pin, a screw, a draw latch, a cable tie, and/or a hook and loop fastener). The tissue anchors may be configured to adhere to the body via an adhesive layer, by piercing, and/or by grasping.

An embodiment can provide a system for approximating tissue includes a stabilizing member (*e.g*., a flexible strip, tape, and/or flexible sheet) and a plurality of anchors configured to be attached to the stabilizing member in a selected relative arrangement. The stabilizing member and/or the plurality of anchors are configured to adhere to a body, and the anchors are configured to approximate tissue of the adhered body upon movement of selected ones of the anchors towards others of the anchors. The system may further include a traction member configured to draw the selected ones of the anchors towards others of the anchors, (such as those described herein, which may be biodegradable or nonbiodegradable). The plurality of anchors may be affixed to the stabilizing member. The anchors may be configured for pairwise attachment, for example via magnets, via mechanical fasteners (*e.g*., a tongue-in-groove connector, a retaining pin, a screw, a draw latch, a cable tie, and/or a hook and loop fastener), via an adhesive, and/or via van der Waals forces.

Embodiments of such systems can find application in a method of closing a wound includes adhering tissue anchors to tissue on opposing sides of the wound, and approximating the attached tissue by coupling the tissue anchors. Approximating the attached tissue may include bringing tissue on opposing sides of the wound into alignment, and coupling the anchors to maintain the alignment. At least a subset of the tissue anchors may include a tissue-adherent portion and a connector portion, and adhering each of the at least a subset of tissue anchors to tissue may include adhering the tissue-adherent portion to tissue, and connecting the connector portion to the tissue-adherent portion (*e.g*., before or after adhering the tissue-adherent portion to tissue). Coupling the tissue anchors may include slidably coupling the tissue anchors, groupwise coupling the tissue anchors, moving the tissue anchors relatively to within a magnetic coupling range, attaching the tissue anchors together with an adhesive, attaching the tissue anchors together with a hook and loop fastener, attaching the tissue anchors together via van der Waals forces, attaching the tissue anchors together with a mechanical fastener (*e.g*., a tongue-in-groove connector, a retaining pin, a screw, a draw latch, a cable tie, and/or a hook and loop fastener), pairwise coupling the tissue anchors, and/or coupling the tissue anchors with a common tensioning structure such as a suture (*e.g*., a shape-memory suture and/or a biodegradable suture) and/or an elastomer. Adhering tissue anchors may include attaching the tissue anchors to tissue on opposing sides of the wound with an adhesive, by piercing the tissue, and/or by grasping the tissue. The wound may be straight, curved, round, branched, stellate, and/or angled.

Embodiments of such systems can find application in a method of approximating a first region of body tissue and a second region of body tissue includes adhering a first tissue anchor to the first region of body tissue, adhering a second tissue anchor to the second region of body tissue, and approximating the adhered tissue by coupling the first and second tissue anchors. The first tissue anchor may include a tissue-adherent portion and a connector portion, and adhering the first tissue anchors to the first region of body tissue may include adhering the tissue-adherent portion to the first region of body tissue, and connecting the connector portion to the tissue-adherent portion (*e.g*., before or after adhering the tissue-adherent portion to the first region of body tissue), Coupling the first and second tissue anchors may include slidably coupling the first and second tissue anchors, moving the first and second tissue anchors relatively to within a magnetic coupling range, attaching the first and second tissue anchors together with an adhesive, attaching the first and second tissue anchors together with a hook and loop fastener, attaching the first and second tissue anchors together via van der Waals forces, attaching the first and second tissue anchors together with a mechanical fastener (*e.g*., a tongue-in-groove connector, a retaining pin, a screw, a draw latch, a cable tie, and/or a hook and loop fastener), and/or coupling the first and second tissue anchors with a common tensioning structure such as a suture (*e.g*., a shape-memory suture and/or a biodegradable suture) and/or an elastomer. Adhering the first tissue anchor may include attaching first the tissue anchor to the first region of body tissue with an adhesive, by piercing the tissue, and/or by grasping the tissue. The first and second regions of body tissue may have been separated before being approximated, and/or they may have been unconnected before being approximated. The method may further include cutting an incision between the first and second regions of body tissue before approximating the first and second regions of body tissue. Approximating the adhered tissue may include applying traction to at least one of the regions of body tissue.

Embodiments of such systems can find application in a method of performing surgery includes adhering tissue couplers to tissue on opposing sides of a planned incision site for a body, cutting an incision between the adhered tissue couplers, accessing the interior of the body via the incision, and closing the incision by coupling the tissue couplers. Adhering the tissue couplers may include adhering a stabilizing member to the body, in which case the tissue couplers may be attached to the stabilizing member at the time that it is adhered to the body, or the method may further include attaching the tissue couplers to the stabilizing member. The stabilizing member may include fiducials that indicate a path for the planned incision. Cutting an incision may include making an opening in skin, in a blood vessel, in muscle tissue, in facial planes, in dura, in dermis, in a visceral tube (*e.g*., an esophagus, a trachea, a respiratory tract, a stomach, a small intestine, a large intestine, a rectum, a ureter, and/or a vas deferens), and/or in bone, and may include cutting with a tool such as a scalpel, cauter, trocar, needle, drill, curette, and/or laser. The incision may be straight, curved, round, branched, stellate, and/or angled. Accessing the interior of the body via the incision may include performing surgery in the body. Closing the incision may include applying a traction member such as a suture to the tissue anchors, coupling the tissue couplers manually, and/or coupling the tissue couplers automatically.

Embodiments of such systems can find application in a method of preparing a body for surgery includes adhering tissue anchors to tissue on opposing sides of a planned incision site on a body. The tissue anchors are configured to be coupled in a configuration that approximates tissue. The tissue anchors may be attached to a stabilizer in a predetermined relative alignment. The stabilizer may include a fiducial that indicates a path for the planned incision, The stabilizer may adhere to the body, and may be a flexible sheet or a tape. The method may further include opening the body along the planned incision site, and may also include closing the body by coupling the tissue anchors.

Embodiments of such systems can find application in a method of attaching an implant to body tissue includes attaching a first anchor to the body tissue, attaching a second anchor to the implant, and attaching the implant to the body tissue by coupling the first anchor and the second anchor. The first anchor may include a tissue-adherent portion and a connector portion, and attaching may include adhering the tissue-adherent portion to the body tissue, and connecting the connector portion to the tissue-adherent portion (*e.g*., before or after attaching the tissue-adherent portion to the body tissue). Coupling the first and second anchors may include slidably coupling the anchors, moving the anchors relatively to within a magnetic coupling range, attaching the anchors with an adhesive, attaching the anchors with a mechanical fastener (*e.g*., a tongue-in-groove connector, a retaining pin, a screw, a draw latch, a cable tie, and/or a hook and loop fastener), attaching the anchors via van der Waals forces, coupling the anchors with an approximation mechanism that joins the anchors in a defined spatial arrangement, such as a filament configured to maintain a tensile force tending to resist relative separation of the first and second anchors (*e.g*., a suture which may be optionally biodegradable and/or may contain a shape-memory material, an elastomer, a cord, and/or a cable tie). The first and second anchors may be threaded on the filament. The implant may include tissue, such as an organ for transplant, in which case the second anchor may optionally be attached before removal of the organ from a donor.

With respect to various embodiments described herein, the surfaces are tissue. It will be understood by those having skill in the art with knowledge of the present disclosure, that the systems and methods described herein can be utilized to approximate or maintain the approximation of two surfaces, at least one of which includes tissue. For ease of reading, the embodiments herein that describe two tissue surfaces are readily adaptable to approximating, or maintaining the approximation of, a tissue surface and a non-tissue surface. The various anchors described herein can be configured to adhere to non-tissue surfaces using the same or similar mechanisms that are described for adhering to tissue, for example by adhesive, surface penetration, friction and/or by any other means.

In addition to the systems and methods described herein for approximating or maintaining the approximation of two tissue surfaces surrounding an incision, it will be understood by those having skill in the art with knowledge of the present disclosure, that the systems and methods described herein can be utilized to approximate or maintain the approximation of surfaces surrounding any opening of tissue. Such an opening may be an incision or a wound. In addition, the systems and methods described herein may be used for approximating or maintaining the approximation of tissue surfaces which may be the same tissue type or they may be distinct. The tissues to be approximated may be naturally separated or may be separated due to an injury or other condition. It will be understood by those having skill in the art with knowledge of the present disclosure that the systems and methods described herein can be utilized to approximate, or maintain the approximation of, tissues such as, by way of non-limiting example, a blood vessel and a muscle tissue, or muscle tissue and bone tissue, ligament tissue to bone tissue, bone tissue to bone tissue, etc.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIER DESCRIPTION OF THE FIGURES

Embodiments are described hereinafter, by way of example only, with reference to the drawings.
**FIG. 1** is a schematic of an incision being closed with a suture and a set of tissue anchors.
**FIG. 2** is a schematic of several different anchor embodiments.
**FIG. 3** is a schematic of an incision being closed with a securing member and a set of tissue anchors.
**FIG. 4** is a schematic of anchors arranged on a stabilizing member.
**FIG. 5** is a schematic of several different embodiments of multi-part couplers.
**FIG. 6** is a schematic of several different multi-part anchor embodiments.
**FIG. 7** is a schematic of a two-part trocar for use in closing the fascia in a laparoscopic procedure.
**FIG. 8** is a schematic of fascia being closed with a suture and a set of tissue anchors.
**FIG. 9** is a flow chart of a method of closing a wound.
**FIG. 10** is a flow chart of a method of performing surgery,
**FIG. 11** is a flow chart of a method of preparing a body for surgery.
**FIG. 12** is a schematic of a computer-implemented system for determining placement of tissue anchors.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the claimed subject matter.

As used herein, the term "biocompatible" means a material the body generally accepts without a significant immune response/rejection or excessive fibrosis. In some embodiments, some immune response and/or fibrosis is desired. In other embodiments, vascularization is desired. In still other embodiments, vascularization is not desired. Biocompatible materials include, but are not limited to, synthetic organic materials such as clinically used nonbiodegradable and biodegradable and bioresorbable polymers including polyglycolide, optically active and racemic polylactides, polydioxanone, and polycaprolactone, polymers under clinical investigation including polyorthoester, polyanhydrides, and polyhydroxyalkanoate, early stage polymeric biomaterials including poly(lactic acid-co-lysine), and shape memory polymers (*e.g*., block copolymers of oligo(ε-caprolactone)diol and crystallisable oligo(p-dioxanone)diol, as described in Lendlein, et al., "Biodegradable, elastic shape-memory polymers for potential biomedical applications," Science, 296(5573):1673-1676 (2002).

As used herein, "biodegradable" materials include materials that at least partially resorb into the body or otherwise break down over time, while "nonbiodegradable" materials include those that maintain substantial mechanical integrity over their lifetime in a body. Such "biodegradable" or "nonbiodegradable" materials are well known to those having skill in the art. In general, the anchors, couplers, traction members, securing members, tensioning members, stabilizing members, and other components described herein may be either biodegradable or nonbiodegradable, or may include both biodegradable and nonbiodegradable components. In some embodiments, these elements will be biocompatible, while in other embodiments, they may be partially or fully constructed from nonbiocompatible materials.

As used herein, "antimicrobial" materials include materials that have the capacity to inhibit the growth of or destroy pathogens, including but not limited to bacteria, fungi, and viruses. Such antimicrobial materials are well known to those having skill in the art and may include materials that are coated or impregnated with an antimicrobial agent or wherein the material itself possesses antimicrobial properties.

As used herein, a material having a "therapeutic property" is one that induces or facilitates a desired biological response. Materials having a therapeutic property are well know to those having skill in the art, and include, but are not limited to cell growth promoters, cell growth inhibitors, cytokines, healing promoters, antibiotics, clotting modulators, anti-inflammatories, and anti-scarring agents.

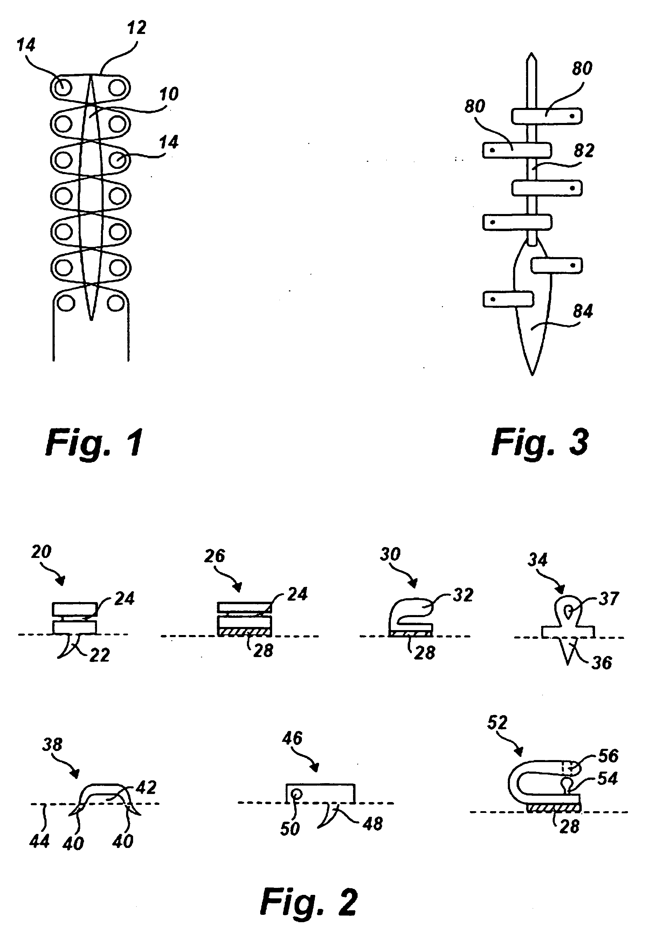

**FIG. 1** illustrates an incision **10** being closed with a suture **12** and a set of tissue anchors **14.** The tissue anchors **14** may be placed in the tissue before or after the incision **10** is made, and may be shaped to receive the suture **12.** To close the incision **10,** the suture **12** is wound around the anchors **14** as shown and pulled to tighten, approximating the body tissue in the region of the anchors **14.** In some embodiments, the edges of the incision may be brought together by other means (*e.g.*, manually by the surgeon), and the suture **12** may be used to maintain the approximation of the edges of the incision. Those of skill in the art of surgery will recognize that there are many possible patterns for placement of the anchors **14** and for winding of the suture **12,** and will be able to select an appropriate configuration for any particular patient and incision. For example, the crossed suture **12** shown in **FIG. 1** may not be desirable in all cases, and may be replaced by a suture winding that does not cross itself, such as a configuration (not shown) in which discrete sutures draw anchors **14** together pairwise across the incision **10,** or a single suture arranged in a serpentine pattern. In other embodiments, it may not be desirable to place all anchors **14** at the same distance from incision **10,** or to place the anchors **14** at regular intervals as shown in **FIG. 1****.** For example, an irregular pattern or a pattern with localized concentrations of anchors **14** may be appropriate for locations having differential topographies, tissue types, expected movement ranges, stresses, or contact with surfaces, such as bandages, supports, clothing, or similar. The number and placement of the anchors **14** will also vary with the incision type, with fewer anchors **14** typically (but not always) being applied for smaller incisions. While **FIG. 1** illustrates an incision **10** being closed, a similar arrangement may be used to close an accidental wound or to draw tissue into a desired configuration (*e.g*., in a face lift or other cosmetic procedure, or in a bladder suspension), or to attach tissue to an implanted device or other object (*e.g.*, an organ for transplant) in a body. While **FIG. 1** illustrates a straight incision **10,** in other embodiments, the opening to be closed by the anchors may be curved, round, branched, stellate, and/or angled (e.g., in a sawtooth configuration).

**FIG. 2** shows a variety of anchor configurations that may be used with a suture to close an incision as shown in **FIG. 1****.** Anchor **20** includes a piercing structure **22** for placement in a body tissue, and a groove **24** to receive a suture. Anchor **26** also includes a groove **24** to receive a suture, but is adhered to the tissue via an adhesive layer **28.** Anchor **30** is adhered to the tissue with an adhesive layer, and includes a hook **32** about which a suture may be looped. Anchor **34** includes a piercing structure **36** of a slightly different shape from that of anchor **20,** and also includes an eyelet **37** through which a suture may be threaded. The piercing structure **36** may allow the anchor to be rotated, either manually or through the natural pulling action of a threaded suture. Anchor **38** includes two piercing prongs **40** like a staple, and creates an opening **42** through which a suture may be passed in cooperation with the underlying tissue **44.** In some embodiments, this anchor may be pushed further into the tissue in a way that prevents movement of the suture, for example after the incision has been closed. Anchor **46** includes a piercing structure **48** and an eyelet **50,** the eyelet **50** being disposed distal from the piercing structure **48** and along the surface of the body tissue. In some embodiments, the eyelets **50** of adjacent anchors **46** may be aligned as the tissue is closed. Anchor **52** includes a vertical post **54** and channel **56** allowing it to be snapped closed, for example after a suture has been threaded around it. In some embodiments, this closure may be reversible, while in others, it may be irreversible. In some embodiments, closure of the anchor 52 may restrict sliding of the suture, while in other embodiments, the suture may be able to slide through the anchor **52** after closure.

The specific structures of anchors shown in **FIG. 2** shall not be interpreted to limit the shape or design of the anchors described and claimed herein. By way of non-limiting example, the piercing structure **22** of anchor **20** may be used in place of the adhesive layer **28** shown with anchor **30;** the eyelet **50** of anchor **46** may be used with the adhesive layer **28** shown with anchor **30** or with the piercing structure **36** shown with anchor **34.** Various combinations of the piercing structures shown, as well as those not shown but known to those of skill in the art, can be used with any suture-holding structure or any other securing member or mechanism.

**FIG. 3** shows another embodiment, in which anchors **80** are secured via insertion of a securing member **82,** which is a conformable rod in the illustrated embodiment. The illustrated anchors **80** are similar to the anchors **46 of** **FIG. 2****,** including a piercing structure (not visible in **FIG. 3****)** and an eyelet through which conformable rod **82** may be inserted. As shown, the rod **82** is partially inserted through the anchors **80,** so that the incision **84** is partially closed. In other embodiments, the anchors **80** or the securing member **82** may include other attachment structures, such as hooks, mating surfaces (to which adhesive may optionally be applied), and/or mechanical fasteners (*e.g*., hook and loop fasteners, draw latches, screws, etc.). By way of non-limiting example, securing member **82** may include a series of hooks configured to receive anchors **80;** anchors **80** may include hooks configured to attach to securing member **82** (which may optionally include predetermined attachment points for anchors **80);** securing member **82** may include snap fittings into which mating portions of anchors **80** may be inserted; anchors **80** and securing member **82** may include holes or other areas configured for attachment of screws or other fasteners that secure anchors **80** and securing member **82** together. In embodiments in which the securing member **82** is conformable, it may be conformed to match the shape of an incision, or it may be conformed before or after insertion in order to apply a mechanical force to tissue in order to reshape it (*e.g*., in cosmetic surgery). In some embodiments, the process of inserting securing member **82** may bring the anchors **80** together, while in other embodiments, the edges of the incision **84** may be brought into alignment before the securing member **82** is deployed.

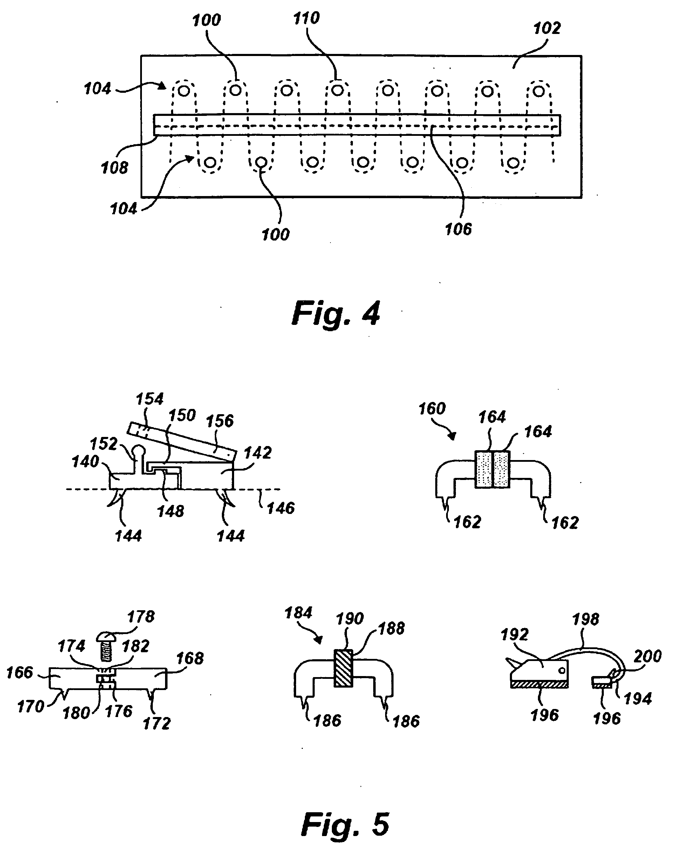

**FIG. 4** **is** a schematic of anchors **100** arranged on a stabilizing member **102.** In the embodiment shown, the stabilizing member **102** includes a flexible tape base designed to adhere to the tissue of interest. The anchors are arranged in parallel rows **104** on opposite sides of a planned incision site **106.** In some embodiments, the flexible tape base may be placed on the patient prior to making the incision. The illustrated embodiment includes an opening **108** along the planned incision site **106,** but other embodiments may omit the opening. The anchors **100** may adhere to the stabilizing member **102,** which in turn adheres to the tissue of interest, via an adhesive, or they may include mechanical fasteners or other structures to facilitate their attachment to tissue (*e.g*., piercing structures such as those shown in anchors **20, 34, 38, 46** of **FIG. 2****).** In one method of use, the stabilizing member **102** is placed on the body with opening **108** positioned at the planned incision site **106.** The incision is made, and surgery is performed on the body via the incision. At the conclusion of the surgery, a suture is threaded around the anchors **100** along serpentine path **110,** and tightened to draw the anchors **100** together, thereby closing the incision (in some embodiments, the incision may be closed by other means, and the suture may maintain the closure). In other embodiments, opening **108** may be omitted, and the incision performed through the stabilizing member **102,** or the stabilizing member **102** may be placed after the incision is made (*e.g*., after the surgery is completed). In some embodiments, the stabilizing member **102** may be applied to a wound (*e.g*., an accidental wound). Rather than a suture, the incision may be closed by application of a securing member as described above in connection with **FIG. 3****,** or by direct connection of couplers as described below in connection with **FIG. 5****.** The stabilizing member **102** may be placed on the skin, or on other tissue such as muscular or vascular tissue.

**FIG. 5** shows several different embodiments of couplers that may be connected without the use of a tensioning member or a securing member as described above. In some embodiments, a specialized or general purpose tool may be used to connect anchors together. Couplers **140, 142** include piercing structures **144** that secure the couplers to underlying tissue **146.** Coupler **140** includes a temporary alignment pin **148** configured to mate with a corresponding alignment groove **150** on coupler **142.** In addition, coupler **140** includes a permanent (or, optionally, semipermanent) retaining pin **152** configured to mate with a channel **154** in hinged connector **156** on coupler **142.** In one method of use, the couplers **140, 142** may be secured to tissue with temporary alignment structures **148, 150** connected. The temporary alignment structures **148, 150** may then be disconnected to permit access to an incision site, for example to open an incision after the couplers **140, 142** have been placed. Upon closing, both temporary alignment structures **148, 150** and permanent retaining structures **152, 154** may be connected, permanently (or, optionally, semipermanently) closing the incision while maintaining the alignment of underlying tissue.

Couplers **160** include piercing structures **162,** and permanent magnets **164.** In use, these couplers may be placed on either side of a wound or a planned incision, and optionally rotated to increase the distance between permanent magnets **164** during access to the wound. Upon closing, the couplers **160** may be rotated (if necessary) to align the magnets, and brought into proximity to magnetically adhere them together, securing the underlying tissue. Couplers **166, 168** include piercing structures **170, 172** for securing them to tissue. A groove **174** in coupler **166** mates with a tongue **176** in coupler **168 to** couple the couplers. This connection can be reversibly or irreversibly secured by insertion of a screw **178** through channels **180, 182** in the couplers **166, 168.** Couplers **184** include piercing structures **186,** and matable surfaces **188.** In use, these couplers may be placed on either side of a wound or a planned incision, and optionally rotated to orient the matable surfaces away from the work area. Upon closing, the couplers **184** may be rotated (if necessary) to align the matable surfaces, which may then be secured together with adhesive **190.** Couplers **192, 194** include adhesive **196** for attachment to tissue (or to a stabilizing member, not shown, or other mechanism for attachment to tissue). Coupler **192** includes latch arm **198,** which engages keeper **200** on coupler **194** to form a draw latch assembly. Latch arm **198** may be rotated away from the work area during surgery, and subsequently engaged to close an underlying incision.

While the couplers illustrated in **FIG. 5** are generally illustrated for coupling in pairwise configurations, in other embodiments, couplers may cooperate in larger groups to close incisions or other wounds. For example, couplers may be arranged in a "zipper" configuration to close a wound along its length. Such an arrangement may include a specialized or general-purpose coupling tool (*e.g*., a zipper pull) to connect couplers together and/or to separate them.

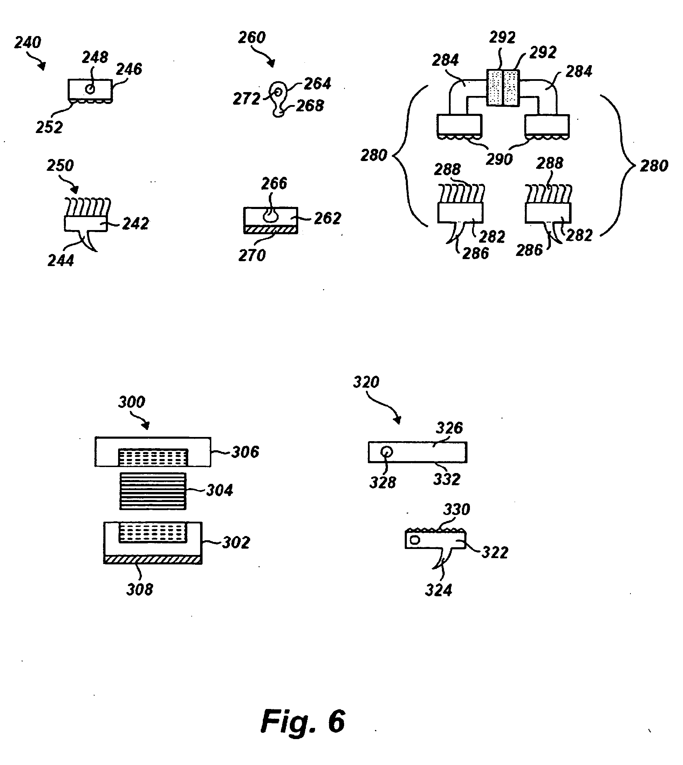

**FIG. 6** is a schematic of several different multi-part anchor embodiments. Each embodiment includes a portion that adheres to tissue, and a portion that engages a suture, a stabilizing member, another anchor, or another closing mechanism. Anchor **240** includes a tissue adherent portion **242,** which is configured to adhere to tissue via piercing mechanism **244,** and connector portion **246,** which is configured to engage a suture via opening **248.** The tissue adherent portion **242** and the connector portion **246** are configured to be connected together via hook-and-loop fasteners **250, 252** (*e.g*., VELCRO™). Anchor **260** includes a tissue adherent portion **262** and a connector portion **264,** which are configured to snap together via mechanical fasteners **266, 268.** Tissue adherent portion **262** includes an adhesive layer **270** configured to adhere to tissue. Connector **264** includes an eyelet **272** configured to receive a suture (not shown). In some embodiments, mechanical fasteners **266, 268** may be configured to form a rotatable connection, which may facilitate alignment of a suture. In either embodiment of anchors **240** or **260,** connector portions **246** or **264** may optionally be pre-threaded onto a suture or a stabilizing member before they are connected to their respective tissue adherent portions **242** or **262,** or they may be connected to their respective tissue adherent portions **242** or **262** and subsequently threaded with a suture or stabilizing member.

Anchors **280** each include a tissue adherent portion **282** and a connector portion **284.** The tissue adherent portions **282** are configured to adhere to tissue via piercing structures **286.** Connector portions **284** are configured to attach to tissue adherent portions **282** via hook-and-loop fasteners **288** and **290** (*e.g*., VELCRO™). Connector portions **284** are also configured to engage one another via magnets **292.** In one method of use, tissue adherent portions **282** may be placed on opposing sides of an incision site, before or after cutting the incision. Upon closing, connectors **284** may be connected to tissue adherent portions **282** and their respective magnets **292** engaged (before or after connection to tissue adherent portions **282),** thereby closing the incision.

Anchor **300** is a three-part anchor, including a tissue adherent portion **302,** a first connector portion **304** configured to screw into tissue adherent portion **302,** and a second connector portion **306** configured to screw onto connector portion **304.** In one method of use, a plurality of tissue adherent portions **302** are adhered to tissue via adhesive layers **308,** for example before an incision is made in the tissue. When it is desired to close the opening, first connector portions **304** are screwed into each respective tissue adherent portion **302.** At this point, a suture or other tensioning member (not shown) may be wound about connector portions **304.** In other embodiments, second connector portions **306** may be partially or fully screwed onto their respective first connector portions **304** before winding or before tightening of the tensioning member. In some embodiments, once the tensioning member has been tightened sufficiently to close the incision, second connector portions **306** may be further screwed onto first connector portions **304,** thereby clamping the tensioning member between tissue adherent portions **302** and second connector portions 306, thereby inhibiting further movement of the tensioning member.

Anchor **320** includes tissue adherent portion **322,** which adheres to tissue via piercing structure **324,** and connector portion **326,** which includes eyelet **328.** Tissue adherent portion **322** and connector portion **326** are configured to attach to one another via van der Waals forces. In the illustrated embodiment, surface **330** includes nanotubes that adhere to flat surface **332** when they are placed in contact (see, *e.g.,* Yurdumakan, et al., "Synthetic gecko foot-hairs from multiwalled carbon nanotubes," Chem. Commun., 2005:3799-3801). In this embodiment, eyelet **328** is located at a distal end of tissue adherent portion **322** when tissue adherent portion **322** and connector portion **326** are attached together. In some embodiments, a straight (or shaped) stabilizing element (not shown) may be threaded through eyelets **328** of a plurality of anchors **320** on opposing sides of a wound, for example in the configuration illustrated in **FIG. 3****.**

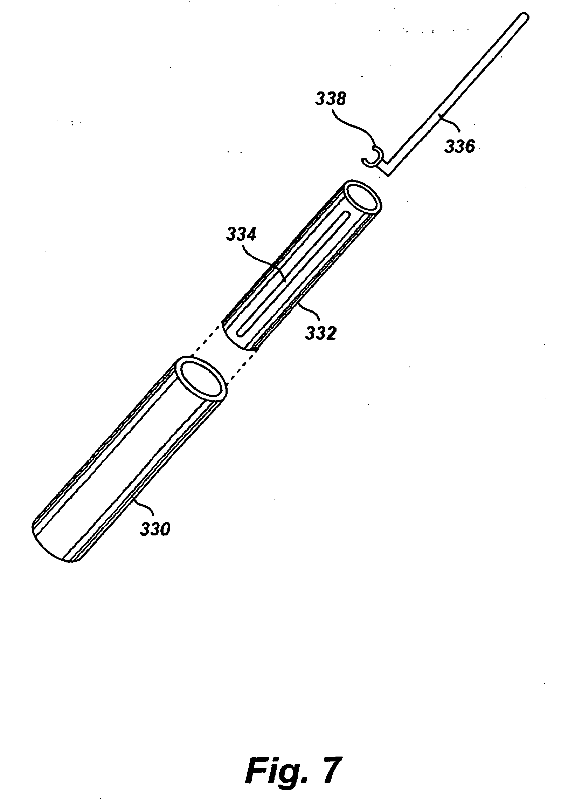

**FIG. 7** illustrates a two-part trocar for use in laparoscopic procedures. The trocar includes a first cylinder **330** having solid walls, and a second cylinder **332** having one or more longitudinal slots **334.** As shown, the second cylinder **332** is sized to fit snugly within first cylinder **330.** In other embodiments, the outer diameter of second cylinder **332** may be smaller than the inner diameter of first cylinder **330,** producing a loose fit between the cylinders. In still other embodiments, the second cylinder **332** may be sized to fit over first cylinder **330,** with either a loose or a snug fit. In still other embodiments, the first cylinder **330** may be eliminated. In such embodiments, if it is necessary to insufflate the underlying body cavity, it may be desirable that a mechanism for sealing slots **334** be integrated into second cylinder **332** in order to maintain pressure within the cavity.

In one method of use, first cylinder **330** is inserted into a body cavity (*e.g*., the abdominal cavity), using a round cutter (not shown) to penetrate the cavity wall. Second cylinder **332** may be integral with first cylinder **330** during insertion, or may be inserted into (or around) first cylinder **330** previously or subsequently, either before or after a laparoscopic procedure is performed. For example, the first cylinder **330** may be inserted as a conventional trocar, and a laparoscopic procedure may be performed. Subsequent to the procedure, but before closing, second cylinder **332** is then inserted into first cylinder **330,** and first cylinder **330** is fully or partially retracted from the body. An anchor placement device **336,** loaded with anchor **338** is then inserted into second cylinder **332.** As shown, the anchor is a split ring, but any of the anchor configurations described herein may be used. In the illustrated embodiment, anchor **338** includes a shape memory alloy. The anchor **338** is inserted through the slot **334** to contact opposing sides of the fascia, and the shape memory phase change is triggered (*e.g.*, by local heating), closing the split ring and piercing the fascia. Multiple anchors **338** may be placed, either using multiple slots **334** or by rotating second cylinder **332** in order to access different positions along the circumference of the fascial opening. Once the anchors **338** have been placed, second cylinder **332** may be fully or partially withdrawn from the opening.

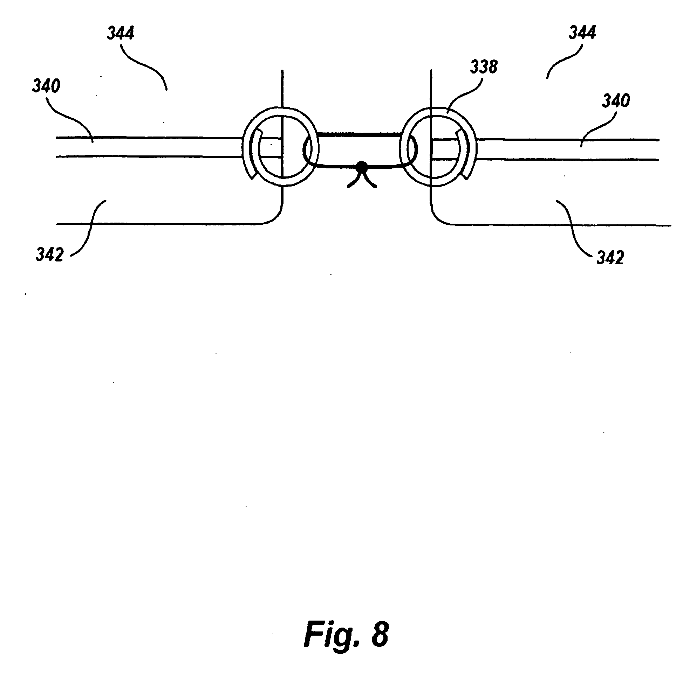

**FIG. 8** illustrates two split ring anchors **338** which have pierced the fascia **340** on either side of a round laparoscopic incision. As shown, the anchors **338** also at least partially penetrate peritoneum **342** and fatty tissue **344.** Anchors **338** are connected by a suture **346.** The suture may be threaded before or after removal of cylinders **330, 332.** Tension may be applied to suture **346** to close the fascia, for example after cylinders **330, 332** have been removed from the incision. In the illustrated embodiment, the suture connects two anchors **338** on opposing sides of the incision, but it will be understood that more anchors may be connected, either by a single suture or other connector looped through all of them, or by a series of pairwise connections (or other connections of smaller subsets of the placed anchors). Tissue anchors may be analogously used to close other layers such as the peritoneum, the muscle layers, and/or the skin. While split-ring anchors **338** have been illustrated in **FIG. 7** and **FIG. 8**, other anchor configurations may be more or less desirable for any particular tissue type and geometry. In some configurations, a suture or other tensioning device may be prethreaded onto tissue anchors, or the anchors may be configured to couple to one another without use of a tensioning device.

In general, the anchors, couplers, traction members, securing members, tensioning members, stabilizing members, and other components described herein may be adjustable or selectively controlled, for example to loosen tension as a joint heals and becomes more flexible or to permit expansion of skin prior to reconstructive surgery or removal for a graft. In particular, any of these components may form a part of or be configured to cooperate with the adjustable implants described in co-pending and commonly owned U.S. Application Nos. 11/710,591, filed February 22, 2007 and entitled, "CODED-SEQUENCE ACTIVATION OF SURGICAL IMPLANTS," and 11/710,592, filed February 22, 2047 and entitled, "CODED-SEQUENCE ACTIVATION OF SURGICAL IMPLANTS,". Any of these components may also be controllable by changing shape or conformation so that such change results in the approximation of surfaces attached to selected anchors, for example via the use of temperature-sensitive, light-sensitive (*e.g*., ultraviolet light-sensitive), touch-sensitive, elastomeric (*e.g.*, an elastomer that is configured to secure each anchor and can reconfigure in a way to approximate surfaces attached to the anchors), or remotely controllable mechanisms.

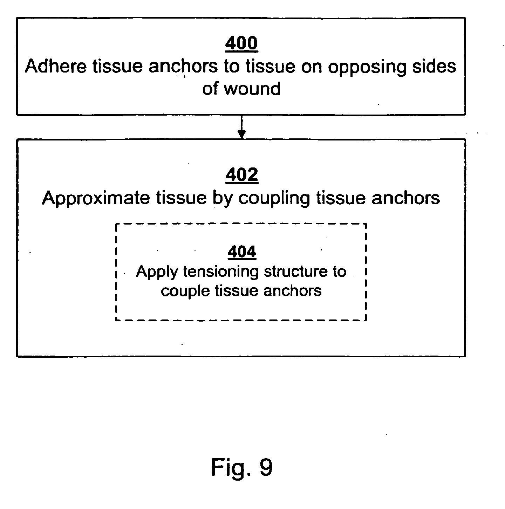

**FIG. 9** is a flow chart illustrating a method of closing a wound. The method includes adhering tissue anchors (*e.g*., anchors such as but not limited to those described in **FIG. 2****,** **FIG.** 5, and/or **FIG. 6****)** to tissue on opposing sides of a wound, **400**, and approximating the tissue by coupling the tissue anchors, **402.** For example, the tissue anchors may be coupled via a tensioning element such as a suture, **404.**

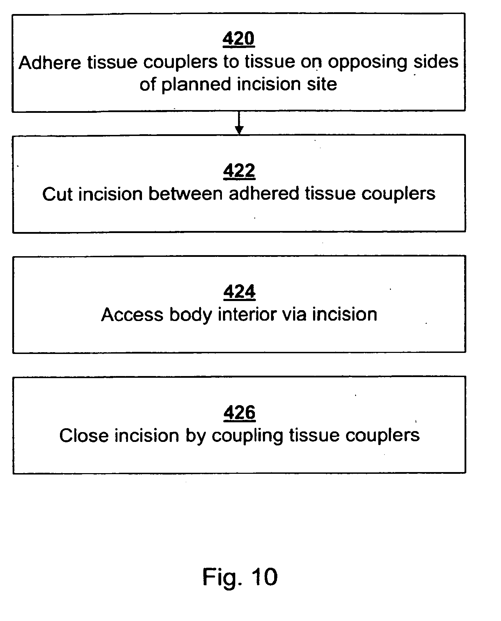

**FIG. 10** is a flow chart illustrating a method of performing surgery. The method includes adhering tissue couplers (*e.g*., couplers such as but not limited to those described in **FIG. 2****,** **FIG. 5****,** and/or **FIG. 6****)** to tissue on opposing sides of a planned incision site, **420,** cutting an incision between the adhered tissue couplers, 422, accessing the interior of the body via the incision (*e.g*., to perform a surgical procedure), **424,** and closing the incision by coupling the tissue couplers, **426.** The incision may be, for example, a straight incision, a curved incision, or a round incision (*e.g*., a round cut such as that made by a trocar). In some embodiments, couplers may be coupled together manually, while in other embodiments, couplers may be coupled together automatically. In some embodiments, the surgery may be endoscopic.

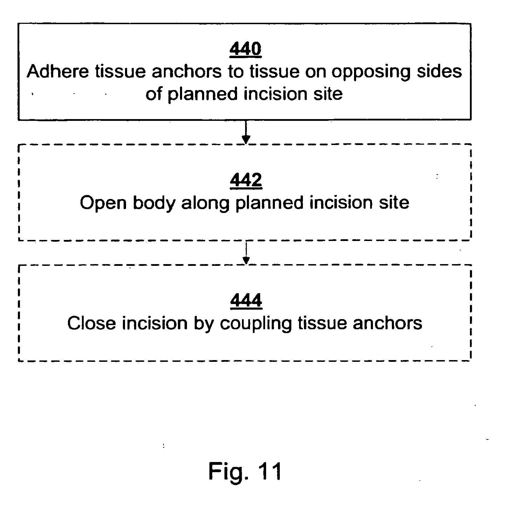

**FIG. 11** is a flow chart illustrating a method of preparing a body for surgery. The method includes adhering tissue anchors to tissue, **440,** on opposing sides of a planned incision site. The method may optionally also include opening the body along the planned incision site, **442,** and/or closing the incision by coupling the tissue anchors, **444.** The incision may be, for example, a straight incision, a curved incision, or a round incision (*e.g*., a round cut such as that made by a trocar).

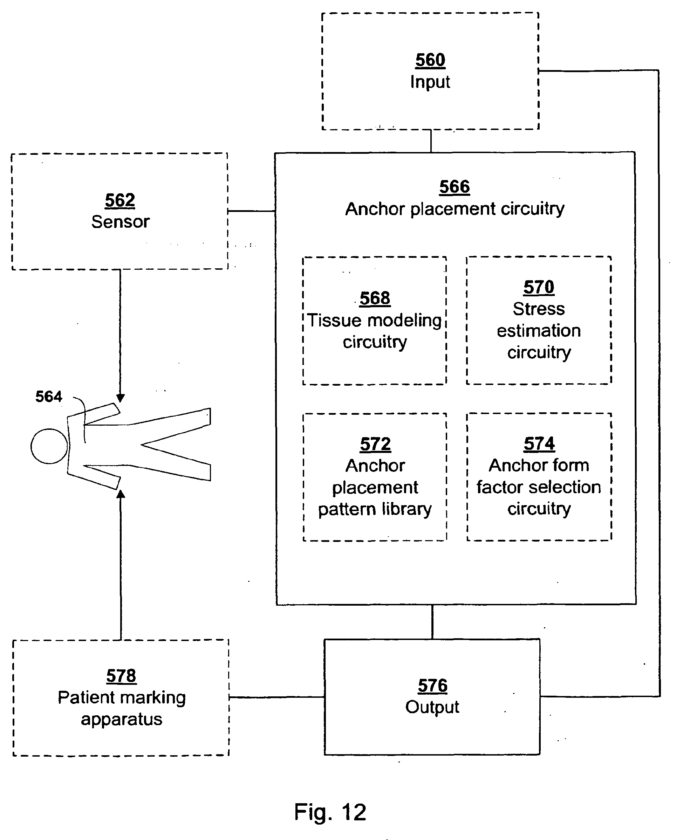

**FIG. 12** illustrates a system for determining placement of tissue anchors (or other suture attachments) for closing an incision. The system may include an input device **560** (*e.g*., a mouse, keyboard, touchscreen, or other machine input system), configured to allow a surgeon to specify a surgery type and/or an incision location. It may further include a sensor **562** that measures one or more physiological parameters of a patient **564** upon whom surgery will be performed. For example, the sensor **562** may include an imaging device that maps the position of organs or other physiological structures that may be taken into account in closing an incision, or it may be a reader (*e.g*., an optical reader) that senses a planned incision location that a surgeon has marked on the body of patient **564.** The input device **560** and/or the sensor **562** may communicate information about the body of patient **564** and/or about the planned surgery to anchor placement circuitry **566.** Anchor placement circuitry **566** may include various subcircuits or subroutines, including but not limited to tissue modeling circuitry **568,** stress estimation circuitry **570,** anchor placement pattern library **572,** and/or anchor form factor selection circuitry **574.**

Tissue modeling circuitry **568** may include circuitry configured to build a computer-based model (*e.g*., a finite element model and/or an analytical model) of the tissue of the patient **564,** for example including specific measurements of sensor **562** and/or physiological or other parameters specified using input device 560. This computer-based model may be used to determine suggested placement for tissue anchors, for example by calculation of the expected response of tissue to particular anchor configurations, and/or by application of stored heuristic rules for expected tissue response. Stress estimation circuitry 570 may be configured to determine expected stresses on anchors and/or on tissue for particular anchor configurations, or it may include optimizing circuitry designed to determine an optimum anchor configuration for a specified design goal. Anchor placement pattern library 372 may include stored configurations of anchors that have been specified by an operator, previously calculated, or otherwise determined. Other portions of the anchor placement circuitry **566** (*e.g*. tissue modeling circuitry **568** and/or stress estimation circuitry **570)** may use the anchor placement pattern library **572** to generate initial placement patterns for calculation, including as a starting point for optimization routines. Anchor form factor selection circuitry **574** may store information about the different form factors of different anchors (such as but not limited to those described herein, *e.g*., in **FIG. 2****,** **FIG. 5****,** and/or **FIG. 6****),** and may further include information about available sizes and mechanical performance of different anchors. It may further include circuitry configured to select a suggested anchor or group of anchors for the particular surgery planned for patient **564.**

The system further includes an output device **576** (*e.g*., a monitor, a printer, a bar code printer, and/or a controller for a patient marking apparatus **578),** which may produce a machine-readable and/or a human-readable output. This output may include calculated anchor placement patterns, tissue responses, anchor stresses, anchor form factors, or other data relevant for placement of anchors during surgery. Output may be iterative and/or interactive, so that a user specifying input via input device **560** may modify input or specify additional inputs in response to output received via output device **576.** For example, output device **576** may output a selection of anchor placement patterns from anchor placement pattern library **572,** and a user may select from among these patterns using input device **560.** Once an anchor placement pattern has been established by anchor placement circuitry **566,** output device **576** may pass data and/or control instructions to a patient marking device **578,** which may temporarily or permanently mark desired anchor placement directly on the patient **564,** or on a tape or other stabilizing member configured to maintain relative anchor locations for attachment to the patient **564.** In other embodiments, the patient marking device may actually place anchors on a stabilizing member for application to a patient **564.**

In a general sense, those skilled in the art will recognize that the various aspects described herein which can be implemented, individually and/or collectively, by a wide range of hardware, software, firmware, or any combination thereof can be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

An embodiment enables tissue to be approximated by adhering anchors to opposing sides of a wound and coupling the anchors together.

Those having skill in the art will recognize that the state of the art of circuit design has progressed to the point where there is typically little distinction left between hardware and software implementations of aspects of systems. The use of hardware or software is generally a design choice representing tradeoffs between cost, efficiency, flexibility, and other implementation considerations. Those having skill in the art will appreciate that there are various vehicles by which processes, systems and/or other technologies involving the use of logic and/or circuits can be effected (e.g., hardware, software, and/or firmware), and that the preferred vehicle will vary with the context in which the processes, systems and/or other technologies are deployed. For example, if an implementer determines that speed is paramount, the implementer may opt for a mainly hardware and/or firmware vehicle. Alternatively, if flexibility is paramount, the implementer may opt for a mainly software implementation. In these or other situations, the implementer may also opt for some combination of hardware, software, and/or firmware. Hence, there are several possible vehicles by which the processes, devices and/or other technologies involving logic and/or circuits described herein may be effected, none of which is inherently superior to the other. Those skilled in the art will recognize that optical aspects of implementations may require optically-oriented hardware, software, and or firmware.

It will be understood by those within the art that, in general, terms used herein, and especially in the appended claims are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of introductory phrases such as "at least one" or "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" *(e.g.,* "an anchor" should typically be interpreted to mean "at least one anchor"); the same holds true for the use of definite articles used to introduce claim recitations. In addition, even if a specific number of an introduced claim recitation *is* explicitly recited, those skilled in the art will recognize that such recitation should typically be interpreted to *mean at least* the recited number (*e.g*., the bare recitation of "two anchors," or "a plurality of anchors," without other modifiers, typically means *at least* two anchors). Furthermore, in those instances where a phrase such as "at least one of A, B, and C," "at least one of A, B, or C," or "an [item] selected from the group consisting of A, B, and C," is used, in general such a construction is intended in the sense one having skill in the art would understand the convention (*e.g*., any of these phrases would include but not be limited to systems that have A alone, B alone, C alone, A and B together, A and C together, B and C together, and/or A, B, and C together). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

While various aspects and embodiments have been disclosed herein, other aspects and embodiments will be apparent to those skilled in the art. The various aspects and embodiments disclosed herein are for purposes of illustration and are not intended to be limiting.

## Claims

1. A system for maintaining the approximation of two surfaces, comprising:
a first anchor (80) and second anchor configured to adhere to a first surface, the first surface including tissue;
a third anchor (80) configured to adhere to a second surface; and
a securing member (82);
**characterised in that** the securing member is configured to operably link and maintain the first, second and third anchors in a predetermined spatial relationship; and
wherein the securing member includes a rod.

2. The system of claim 1, wherein at least one of the first, second and third anchors includes an opening configured to receive the securing member.

3. The system of claim 2, wherein the opening is selected from the group consisting of an eyelet, a channel, and a hook.

4. The system of any preceding claim, wherein the securing member is configured to attach to each of the first, second and third anchors.

5. The system of claim 4, wherein the first anchor includes a first opening, the second anchor includes a second opening, the third anchor includes a third opening, and the securing member is configured to be inserted into the first, the second and the third openings.

6. The system of any preceding claim, wherein the rod includes one or more of a conformable rod, a substantially straight rod, or a rod having a nonlinear shape.

7. The system of any preceding claim, wherein the securing member is integral to at least one of the first, second and third anchors.

8. The system of any preceding claim, wherein the first and second anchors are configured to adhere to the first surface by one or more of an adhesive, by penetrating tissue, by grasping, or by friction.

9. The system of any preceding claim, wherein at least one of the first, second and third anchors includes a surface-adherent portion configured to adhere to a surface; and an engagement portion configured to engage the securing member.

10. The system of claim 9, wherein the surface-adherent portion and the engagement portion are separate or separable.

11. The system of claim 9 or 10, wherein the surface-adherent portion and the engagement portion are integral.

12. The system of any of claims 9 to 11, wherein the surface-adherent portion and the engagement portion are configured to mechanically lock together.

13. The system of any of claims 9 to 11, wherein the surface-adherent portion and the engagement portion are configured to be glued together with an adhesive.

14. The system of any of claims 9 to 13, wherein the engagement portion includes an opening configured to accept at least a portion of the securing member.

15. The system of claim 14, wherein the opening is selected from the group consisting of a loop, a channel, and a hook.

16. The system of any preceding claim, wherein at least one of the first and second anchors includes a material having a therapeutic property selected from the group consisting of cell growth promoters, cell growth inhibitors, cytokines, healing promoters, antibiotics, clotting modulators, anti-inflammatory agents, and anti-scarring agents.

17. The system of any preceding claim, wherein at least one of the first, second and third anchors includes a biocompatible material.

18. The system of any preceding claim, wherein at least one of the first, second and third anchors includes a biodegradable material.

19. The system of any preceding claim, wherein at least one of the first, second and third anchors includes a nonbiodegradable material.

20. The system of any preceding claim, wherein at least one of the first, second and third anchors includes an antimicrobial material.

21. The system of any preceding claim, wherein the second surface includes tissue.

22. The system of any of claims 1 to 20 , wherein the second surface includes an organ for transplant.

23. The system of any of claims 1 to 20, wherein the second surface includes non-tissue.

24. The system of any of claims 1 to 20, wherein the second surface is an implant device.

25. The system of claim 24, wherein the implant device is selected from the group consisting of a stent, a replacement heart valve, a breast implant, a cerebral stimulator, an endosseous implant, a bone growth matrix, an electrode, an aneurysm coil, a cosmetic implant, a vascular graft, an orthopedic implant, a neural implant, allograft, autograft, xenograft, cardiac implant and a graft.

## Patentansprüche

1. System zum Aufrechterhalten der Approximation von zwei Flächen, umfassend:
einen ersten Anker (80) und einen zweiten Anker, die dazu konfiguriert sind, an einer ersten Fläche zu haften, wobei die erste Fläche Gewebe umfasst;
einen dritten Anker (80), der dazu konfiguriert ist, an einer zweiten Fläche zu haften; und
ein Befestigungsglied (82);
**dadurch gekennzeichnet, dass** das Befestigungsglied dazu konfiguriert ist, den ersten, den zweiten und den dritten Anker in einer vorbestimmten räumlichen Beziehung wirkzuverbinden und zu halten; und
wobei das Befestigungsglied eine Stange umfasst.

2. System nach Anspruch 1, wobei der erste und/oder der zweite und/oder der dritte Anker eine Öffnung enthält/enthalten, die dazu konfiguriert ist, das Befestigungsglied aufzunehmen.

3. System nach Anspruch 2, wobei die Öffnung aus der aus einer Öse, einem Kanal und einem Haken bestehenden Gruppe ausgewählt ist.

4. System nach einem vorhergehenden Anspruch, wobei das Befestigungsglied dazu konfiguriert ist, an dem ersten, dem zweiten und dem dritten Anker befestigt zu werden.

5. System nach Anspruch 4, wobei der erste Anker eine erste Öffnung enthält, der zweite Anker eine zweite Öffnung enthält, der dritte Anker eine dritte Öffnung enthält und das Befestigungsglied dazu konfiguriert ist, in die erste, die zweite und die dritte Öffnung eingeführt zu werden.

6. System nach einem vorhergehenden Anspruch, wobei die Stange eine anpassbare Stange und/oder eine im Wesentlichen gerade Stange und/oder eine Stange mit einer nicht linearen Form umfasst.

7. System nach einem vorhergehenden Anspruch, wobei das Befestigungsglied mit dem ersten und/oder dem zweiten und/oder dem dritten Anker integral ist.

8. System nach einem vorhergehenden Anspruch, wobei der erste und der zweite Anker dazu konfiguriert sind, durch einen Klebstoff und/oder durch Eindringen in Gewebe und/oder durch Ergreifen und/oder durch Reibung an der ersten Fläche zu haften.

9. System nach einem vorhergehenden Anspruch, wobei der erste und/oder der zweite und/oder der dritte Anker einen flächenhaftenden Teil enthält/enthalten, der dazu konfiguriert ist, an einer Fläche zu haften; und ein Eingriffsteil dazu konfiguriert ist, das Befestigungsglied in Eingriff zu nehmen.

10. System nach Anspruch 9, wobei der flächenhaftende Teil und der Eingriffsteil getrennt oder trennbar sind.

11. System nach Anspruch 9 oder 10, wobei der flächenhaftende Teil und der Eingriffsteil integral sind.

12. System nach einem der Ansprüche 9 bis 11, wobei der flächenhaftende Teil und der Eingriffsteil dazu konfiguriert sind, mechanisch miteinander verriegelt zu werden.

13. System nach einem der Ansprüche 9 bis 11, wobei der flächenhaftende Teil und der Eingriffsteil dazu konfiguriert sind, mit einem Klebstoff miteinander verklebt zu werden.

14. System nach einem der Ansprüche 9 bis 13, wobei der Eingriffsteil eine Öffnung umfasst, die dazu konfiguriert ist, mindestens einen Teil des Befestigungsglieds aufzunehmen.

15. System nach Anspruch 14, wobei die Öffnung aus der aus einer Schlaufe, einem Kanal und einem Haken bestehenden Gruppe ausgewählt ist.

16. System nach einem vorhergehenden Anspruch, wobei der erste und/oder der zweite Anker ein Material mit einer therapeutischen Eigenschaft aufweist/aufweisen, das aus der aus Zellwachstumsförderern, Zellwachstumshemmern, Zytokinen, Heilungsbeschleunigern, Antibiotika, Gerinnungsmodulatoren, Entzündungshemmern und Mitteln zur Verhinderung von Narbenbildung bestehenden Gruppe ausgewählt ist.

17. System nach einem vorhergehenden Anspruch, wobei der erste und/oder der zweite und/oder der dritte Anker ein biokompatibles Material umfasst/umfassen.

18. System nach einem vorhergehenden Anspruch, wobei der erste und/oder der zweite und/oder der dritte Anker ein biologisch abbaubares Material umfasst/umfassen.

19. System nach einem vorhergehenden Anspruch, wobei der erste und/oder der zweite und/oder dritte Anker ein biologisch nicht abbaubares Material umfasst/umfassen.

20. System nach einem vorhergehenden Anspruch, wobei der erste und/oder der zweite und/oder der dritte Anker ein antimikrobielles Material umfasst/umfassen.

21. System nach einem vorhergehenden Anspruch, wobei die zweite Fläche Gewebe umfasst.

22. System nach einem der Ansprüche 1 bis 20, wobei die zweite Fläche ein Organ zur Transplantation umfasst.

23. System nach einem der Ansprüche 1 bis 20, wobei die zweite Fläche nicht-Gewebe umfasst.

24. System nach einem der Ansprüche 1 bis 20, wobei die zweite Fläche eine Implantatvorrichtung ist.

25. System nach Anspruch 24, wobei die Implantatvorrichtung aus der aus einem Stent, einer Ersatzherzklappe, einem Brustimplantat, einem Hirnstimulator, einem endossalen Implantat, einer Knochenwachstumsmatrix, einer Elektrode, einer Aneurysma-Spirale, einem kosmetischen Implantat, einem Gefäßtransplantat, einem orthopädischen Implantat, einem Neuroimplantat, einem Allotransplantat, einem Autotransplantat, einem Xenotransplantat, einem Herzimplantat und einem Transplantat bestehenden Gruppe ausgewählt ist.

## Revendications

1. Système destiné à maintenir la proximité de deux surfaces, comportant .
une première ancre (80) et une deuxième ancre configurées pour adhérer à une première surface, la première surface comprenant du tissu ;
une troisième ancre (80) configurée pour adhérer à une second surface ; et
un élément (82) de fixation ;
**caractérisé en ce que** l'élément de fixation est configuré pour relier fonctionnellement et maintenir les première, deuxième et troisième ancres dans une relation spatiale prédéterminée ; et
l'élément de fixation comprenant une tige.

2. Système selon la revendication 1, au moins une des première, deuxième et troisième ancres comprenant une ouverture configurée pour recevoir l'élément de fixation.

3. Système selon la revendication 2, l'ouverture étant choisie dans le groupe constitué d'un billet, d'un conduit et d'un crochet.

4. Système selon l'une quelconque des revendications précédentes, l'élément de fixation étant configuré pour s'accrocher à chacune des première, deuxième et troisième ancres.

5. Système selon la revendication 4, la première ancre comprenant une première ouverture, la deuxième ancre comprenant une deuxième ouverture, la troisième ancre comprenant une troisième ouverture et l'élément de fixation étant configuré pour être inséré dans la première, la deuxième et la troisième ouverture.

6. Système selon l'une quelconque des revendications précédentes, la tige comprenant une tige configurable, une tige sensiblement rectiligne et / ou une tige présentant une forme non linéaire.

7. Système selon l'une quelconque des revendications précédentes, l'élément de fixation étant intégré à au moins une des première, deuxième et troisième ancres.

8. Système selon l'une quelconque des revendications précédentes, les première et deuxième ancres étant configurées pour adhérer à la première surface par un adhésif, en pénétrant dans un tissu, par préhension et / ou par frottement.

9. Système selon l'une quelconque des revendications précédentes, au moins une des première, deuxième et troisième ancres comprenant une partie d'adhérence à la surface configurée pour adhérer à une surface ; et une partie d'interaction configurée pour interagir avec l'élément de fixation.

10. Système selon la revendication 9, la partie d'adhérence à la surface et la partie d'interaction étant séparées ou séparables.

11. Système selon la revendication 9 ou 10, la partie d'adhérence à la surface et la partie d'interaction étant intégrées.

12. Système selon l'une quelconque des revendications 9 à 11, la partie d'adhérence à la surface et la partie d'interaction étant configurées pour se verrouiller mécaniquement ensemble.

13. Système selon l'une quelconque des revendications 9 à 11, la partie d'adhérence à la surface et la partie d'interaction étant configurées pour être collées ensemble à l'aide d'un adhésif.

14. Système selon l'une quelconque des revendications 9 à 13, la partie d'interaction comprenant une ouverture configurée pour admettre au moins une partie de l'élément de fixation.

15. Système selon la revendication 14, l'ouverture étant choisie dans le groupe constitué d'une boucle, d'un conduit et d'un crochet.

16. Système selon l'une quelconque des revendications précédentes, au moins une des première et deuxième ancres comprenant un matériau présentant une propriété thérapeutique choisie dans le groupe constitué des activateurs de croissance cellulaire, des inhibiteurs de croissance cellulaire, des cytokines, des facteurs de cicatrisation, des antibiotiques, des modulateurs de coagulation, des agents antiinflammatoires et des agents anti-cicatrices.

17. Système selon l'une quelconque des revendications précédentes, au moins une des première, deuxième et troisième ancres comprenant un matériau biocompatible.

18. Système selon l'une quelconque des revendications précédentes, au moins une des première, deuxième et troisième ancres comprenant un matériau biodégradable.

19. Système selon l'une quelconque des revendications précédentes, au moins une des première, deuxième et troisième ancres comprenant un matériau non biodégradable.

20. Système selon l'une quelconque des revendications précédentes, au moins une des première, deuxième et troisième ancres comprenant un matériau antimicrobien.

21. Système selon l'une quelconque des revendications précédentes, la deuxième surface comprenant du tissu.

22. Système selon l'une quelconque des revendications 1 à 20, la deuxième surface comprenant un organe pour transplantation.

23. Système selon l'une quelconque des revendications 1 à 20, la deuxième surface comprenant un élément non tissulaire.

24. Système selon l'une quelconque des revendications 1 à 20, la deuxième surface étant un dispositif d'implant.

25. Système selon la revendication 24, le dispositif d'implant étant choisi dans le groupe constitué d'un stent, d'une valve cardiaque de remplacement, d'un implant mammaire, d'un stimulateur cérébral, d'un implant endo-osseux, d'une matrice de croissance osseuse, d'une électrode, d'un serpentin pour anévrisme, d'un implant cosmétique, d'une greffe vasculaire, d'un implant orthopédique, d'un implant neural, d'une allogreffe, d'une autogreffe, d'une xénogreffe, d'un implant cardiaque et d'une greffe.
